# EUROPEAN PATENT APPLICATION

(11) **EP 4 767 974 A1**
(43) Date of publication of application: **01.07.2026**
(21) Application number: 26150044.1
(22) Date of filing: 02.01.2026
(51) Int. Cl.: A61B 18/14, A61B 17/32, A61B 18/00

(54) **TECHNOLOGIES FOR NON-CONDUCTIVE TISSUE CLAMP WITH CONDUCTIVE TRACES FOR ENERGY-BASED SURGICAL INSTRUMENTS**

(30) Priority: 31.12.2024 US 202463740947 P; 26.09.2025 US 202519341029
(71) Applicant: Cilag GmbH International, 6300 Zug (CH)
(72) Inventor: SHELTON, IV, Frederick E., Cincinnati, 45242 (US); HARRIS, Jason L., Cincinnati, 45242 (US); MESSERLY, Jeffrey D., Cincinnati, 45242 (US); HARNETT, Vincent, Cincinnati, 45242 (US); DAVE, Vipul, Cincinnati, 45242 (US); ROSE, Daniel, Cincinnati, 45242 (US); KEENAN, Michael, Cincinnati, 45242 (US); PATEL, Sudir, Cincinnati, 45242 (US)
(74) Representative: Carpmaels & Ransford LLP

(57) **Abstract**

A surgical instrument and associated method includes an end effector having an ultrasonic blade, a metallic jaw clamp, and a partially conductive tissue pad coupled to the jaw clamp. The tissue pad comprises a polymeric substrate with a conductive trace formed on the surface of the polymeric substrate. The polymeric substate may be formed from polytetrafluoroethylene (PTFE), and the surface of the PTFE substrate may be treated to improve adhesion of the conductive trace. The tissue pad is erodible during activation of the ultrasonic blade. The conductive trace may be a multilayer laminate. A method for constructing the surgical instrument includes forming the conductive trace on the surface of the polymeric substrate and securing the polymeric substrate to the metallic jaw clamp.

## Description

### CROSS-REFERENCE TO RELATED APPLICATION

This application claims the benefit of and priority to U.S. Patent Application No. 63/740,947, entitled "TECHNOLOGIES FOR NON-CONDUCTIVE TISSUE CLAMP WITH CONDUCTIVE TRACES FOR ENERGY-BASED SURGICAL INSTRUMENTS," which was filed on December 31, 2024, and which is incorporated herein by reference in its entirety.

### TECHNICAL FIELD

The present disclosure relates generally to energy-based surgical instruments and, more particularly, to harmonic and/or electrosurgical surgical instruments.

### BACKGROUND

Energy-based surgical instruments are finding increasingly widespread applications in surgical procedures by virtue of their unique performance characteristics. Depending upon specific device configurations and operational parameters, energy-based surgical instruments can provide both transection of tissue and hemostasis of the tissue by coagulation, which may reduce or otherwise minimize patient trauma. Depending on the particular application, energy-based surgical instruments may utilize different surgical technologies including, for example, ultrasonic and/or electro-surgical (e.g., radio frequency (RF)) technologies.

A typical ultrasonic surgical instrument may include a handpiece containing an ultrasonic transducer and an elongated shaft assembly having a distally mounted end effector to effect the cutting and sealing of tissue. For example, the end effector may include a jaw assembly having an ultrasonic blade and a clamp arm, which may include a non-stick tissue pad or similar bed to receive the ultrasonic blade. In some cases, the elongated shaft assembly may be permanently affixed to the handpiece. In other cases, the elongated shaft assembly may be detachable from the handpiece, as in the case of a disposable shaft assembly or a shaft assembly that is interchangeable between different handpieces. In use, the end effector transmits ultrasonic energy to tissue brought into contact with the ultrasonic blade of the end effector to realize the cutting and sealing action. Such ultrasonic surgical devices may be configured for open surgical use, laparoscopic, and/or endoscopic surgical procedures including robotic-assisted procedures.

Ultrasonic energy cuts and coagulates tissue using temperatures lower than those used in electro-surgical procedures. Vibrating at high frequencies (e.g., 55,500 times per second), the ultrasonic blade denatures protein in the tissue to form a sticky coagulum. Pressure exerted on tissue by the ultrasonic blade surface collapses blood vessels and allows the coagulum to form a hemostatic seal. A surgeon can control the cutting speed and coagulation by the force applied to the tissue by the end effector, the time over which the force is applied, and the selected excursion level of the end effector.

In electro-surgical instruments, one or more electrodes are incorporated into the end effector and configured to apply therapeutic electrical current to the patient's tissue to create a hemostatic seal. In electro-surgical instruments that do not include a harmonic mode (i.e., do not include a harmonic blade), the end effector may be embodied as two clamp arms or jaws. In such embodiments, the electro-surgical instrument may include a separate mechanical knife or blade for cutting the tissue after the creation of the hemostatic seal, which may be incorporated into the elongated shaft attached to the end effector. In bi-polar embodiments, an active electrode have be attached to one of the clamp arms of the end effector and configured to introduce an electrical current into the tissue, which is received by a return electrode attached to the other clamp arm of the end effector (or as the blade itself in embodiments including a harmonic mode). Conversely, in mono-polar embodiments, the return electrode (e.g., a "grounding pad") may be separate from the electro-surgical instrument and located on a different part of the body of the patient. In some embodiments, the electro-surgical instrument may also be configured to apply a sub-therapeutic electrical current to the patient's tissue, which may be used for sensing purposes (e.g., measuring tissue impedance).

Electro-surgery forms hemostatic seals by generating heat in the tissue via the introduced electrical energy, which is embodied as radio frequency ("RF") energy. The particular frequency employed can vary based on the intended use of the electro-surgical instrument within the range of about 100kHz to 1 MHz, although higher frequencies can be employed in some embodiments. Additionally, sub-therapeutic frequencies may be used in some situations for purposes other than hemostatic sealing, such as performing various electrical measurements on the tissue.

It should be appreciated that some energy-based surgical instrument may employ dual or multi-modal technologies for the transection and/or hemostasis of patient tissue. For example, in some cases, an energy-based surgical instrument may include both ultrasonic and electro-surgical capabilities (e.g., by utilizing the ultrasonic blade as an electrode for the electro-surgery mode), which increases the surgical options provided by the surgical instrument to the surgeon.

### SUMMARY

According to an aspect of the present disclosure, a surgical instrument includes an end effector having an ultrasonic blade, a metallic jaw clamp, and a partially conductive tissue pad coupled to the jaw clamp. The tissue pad includes a polymeric substrate with a conductive trace formed on the surface of the polymeric substrate. The conductive trace covers part or all of the polymeric substrate. In some embodiments, the conductive trace is coupled to a radio-frequency electrosurgical generator.

In some embodiments, the surgical instrument further includes a non-conductive tissue stability feature defined on the surface of the polymeric substrate. In some embodiments, the surgical instrument further includes a recess defined in the non-conductive tissue stability feature. The conductive trace is positioned in the recess. In some embodiments, the surgical instrument further includes an adhesion promotion feature defined on the surface of the polymeric substrate. The conductive trace is positioned on the adhesion promotion feature.

In some embodiments, the polymeric substrate comprises polytetrafluoroethylene (PTFE). In some embodiments, the surface of the polymeric substrate is treated to improve adhesion. In some embodiments, the surface of the polymeric substrate is treated by sodium ammonia etching. In some embodiments, the surface of the polymeric substrate is treated by plasma etching or parylene coating. In some embodiments, the polymeric substrate comprises polyetheretherketone (PEEK), polyetherimide (PEI), polyphenylene sulfide (PPS), polyimide (PI) polysulfone (PSU), polypropylene (PP), polystyrene (PS), polyethylene terephthalate (PET), fiberglass (FG), polycarbonate (PC), acrylonitrile butadiene styrene (ABS), or polyvinylchloride (PVC).

In some embodiments, the conductive trace comprises a conductive ink. In some embodiments, the conductive trace comprises a conductive polymer.

In some embodiments, the conductive trace comprises a multi-layer laminate conductive trace. In some embodiments, the multi-layer laminate conductive trace is erodible by the ultrasonic blade without electrically shorting to the ultrasonic blade. In some embodiments, the multi-layer laminate conductive trace has a first thickness. The ultrasonic blade erodes less than the first thickness of the multi-layer laminate conductive trace when activated.

In some embodiments, the end effector further includes a jaw assembly movable between an open state and a closed state. When the jaw assembly is in the closed state, the metallic jaw clamp is clamped against the ultrasonic blade. When clamped, the polymeric substrate contacts the ultrasonic blade and the conductive trace is electrically insulated from the ultrasonic blade. In some embodiments, when the ultrasonic blade is activated the polymeric substrate erodes and the conductive trace remains electrically insulated from the ultrasonic blade.

In some embodiments, the end effector further includes a jaw assembly movable between an open state and a closed state. When the jaw assembly is in the closed state, the metallic jaw clamp is clamped against the polymeric substrate. When clamped the polymeric substrate contacts the ultrasonic blade at a blade contact area. A first portion of the conductive trace extends across the blade contact area of the polymeric substrate and contacts the ultrasonic blade when the metallic jaw clamp is clamped against the polymeric substrate. In some embodiments, when the ultrasonic blade is activated the first portion of the conductive trace erodes, and the conductive trace is electrically insulated from the ultrasonic blade after activation of the ultrasonic blade.

According to another aspect, a method for constructing a surgical instrument includes forming a conductive trace on a surface of a polymeric substrate, wherein the trace covers part or all of the polymeric substrate; and securing the polymeric substrate to a metallic jaw clamp of a surgical instrument. In some embodiments, the method further includes coupling the conductive trace to a radio-frequency electrosurgical generator after securing the polymeric substrate to the metallic jaw clamp.

In some embodiments, the method further includes forming a non-conductive tissue stability feature on the surface of the polymeric substrate. In some embodiments, a recess is defined in the non-conductive tissue stability feature; wherein forming the conductive trace includes forming the conductive trace in the recess. In some embodiments, the method further includes forming an adhesion promotion feature on the surface of the polymeric substrate; wherein forming the conductive trace includes forming the conductive trace on the adhesion promotion feature.

In some embodiments, the polymeric substrate comprises polytetrafluoroethylene (PTFE). In some embodiments, the method further includes treating the surface of the polymeric substrate to improve adhesion; wherein forming the conductive trace comprises forming the conductive trace after treating the surface. In some embodiments, treating the surface includes etching the surface with sodium ammonia. In some embodiments, treating the surface includes plasma etching the surface or coating the surface with parylene. In some embodiments, the polymeric substrate comprises polyetheretherketone (PEEK), polyetherimide (PEI), polyphenylene sulfide (PPS), polyimide (PI) polysulfone (PSU), polypropylene (PP), polystyrene (PS), polyethylene terephthalate (PET), fiberglass (FG), polycarbonate (PC), acrylonitrile butadiene styrene (ABS), or polyvinylchloride (PVC).

In some embodiments, forming the conductive trace on the surface of the polymeric substrate includes printing the conductive trace with a conductive ink. In some embodiments, forming the conductive trace on the surface of the polymeric substrate includes depositing the conductive trace with a conductive polymer.

In some embodiments, forming the conductive trace on the surface of the polymeric substrate includes applying a multi-layer microfluidic reactor to the surface of the polymeric substrate; and flowing the trace onto the surface of the polymeric substrate with the multi-layer fluidic reactor. In some embodiments, the multi-layer microfluidic reactor includes a soft polymeric material, wherein the soft polymeric material comprises polydimethylsiloxane (PDMS) or silicone.

In some embodiments, forming the conductive trace includes forming a multi-layer laminate conductive trace on the surface of the polymeric substrate. In some embodiments, the multi-layer laminate conductive trace is erodible by an ultrasonic blade without electrically shorting to the ultrasonic blade. In some embodiments, the multi-layer laminate conductive trace has a first thickness, and wherein the ultrasonic blade erodes less than the first thickness of the multi-layer laminate conductive trace when activated.

In some embodiments, the method further includes clamping the metallic jaw clamp against an ultrasonic blade, wherein when clamped the polymeric substrate contacts the ultrasonic blade and the conductive trace is electrically insulated from the ultrasonic blade. In some embodiments, when the ultrasonic blade is activated the polymeric substrate erodes and the conductive trace remains electrically insulated from the ultrasonic blade.

In some embodiments, the method further includes clamping the metallic jaw clamp against an ultrasonic blade, wherein when clamped the polymeric substrate contacts the ultrasonic blade at a blade contact area, and wherein a first portion of the conductive trace extends across the blade contact area of the polymeric substrate and contacts the ultrasonic blade. In some embodiments, when the ultrasonic blade is activated the first portion of the conductive trace erodes, and the conductive trace is electrically insulated from the ultrasonic blade after activation of the ultrasonic blade.

### BRIEF DESCRIPTION OF THE DRAWINGS

The detailed description particularly refers to the following figures, in which:
FIG. 1 is a simplified diagram of an embodiment of a system for performing an energy-based surgical procedure;
FIG. 2 is a perspective view of an embodiment of an energy-based surgical instrument of the system of FIG. 1;
FIG. 3 is a side elevation view of a jaw assembly of an end effector of the surgical instrument of FIG. 2 including an ultrasonic blade and in an open state;
FIG. 4 is a side elevation view of the jaw assembly of the end effector of the surgical instrument of FIG. 2 including an ultrasonic blade and in a closed state;
FIG. 5A is a perspective view of another embodiment of the end effector of the surgical instrument of FIG. 2 including an electrode on a lower jaw clamp of the jaw assembly;
FIG. 5B is a perspective view of another embodiment of the end effector of the surgical instrument of FIG. 2 including two jaw clamps, each having an electrode attached thereto;
FIG. 6 is an exploded view of the surgical instrument of FIG. 2;
FIG. 7 is a block diagram of a control circuit of the surgical instrument of FIG. 2;
FIG. 8 is a perspective view of a jaw clamp of the surgical instrument of FIG. 2 including a tissue pad with conductive trace;
FIG. 9 is a cross-sectional view of at least one embodiment of the jaw clamp with tissue pad of FIG. 8;
FIG. 10 is a cross-sectional view of at least one additional embodiment of the jaw clamp with tissue pad of FIG. 8; and
FIG. 11 is a simplified flow diagram of a method for manufacturing a tissue pad and/or jaw clamp of FIGS. 8-10.

### DETAILED DESCRIPTION OF THE DRAWINGS

While the concepts of the present disclosure are susceptible to various modifications and alternative forms, specific illustrative embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

Terms representing anatomical references, such as anterior, posterior, medial, lateral, superior, inferior, distal, proximal, et cetera, may be used throughout the specification in reference to the surgical instruments described herein as well as in reference to the patient's natural anatomy. Such terms have well-understood meanings in both the study of anatomy and the field of surgery. Use of such anatomical reference terms in the written description and claims is intended to be consistent with their well-understood meanings unless noted otherwise.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

The disclosed embodiments may be implemented, in some cases, in hardware, firmware, software, or any combination thereof. The disclosed embodiments may also be implemented as instructions carried by or stored on a transitory or non-transitory machine-readable (e.g., computer-readable) storage medium, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

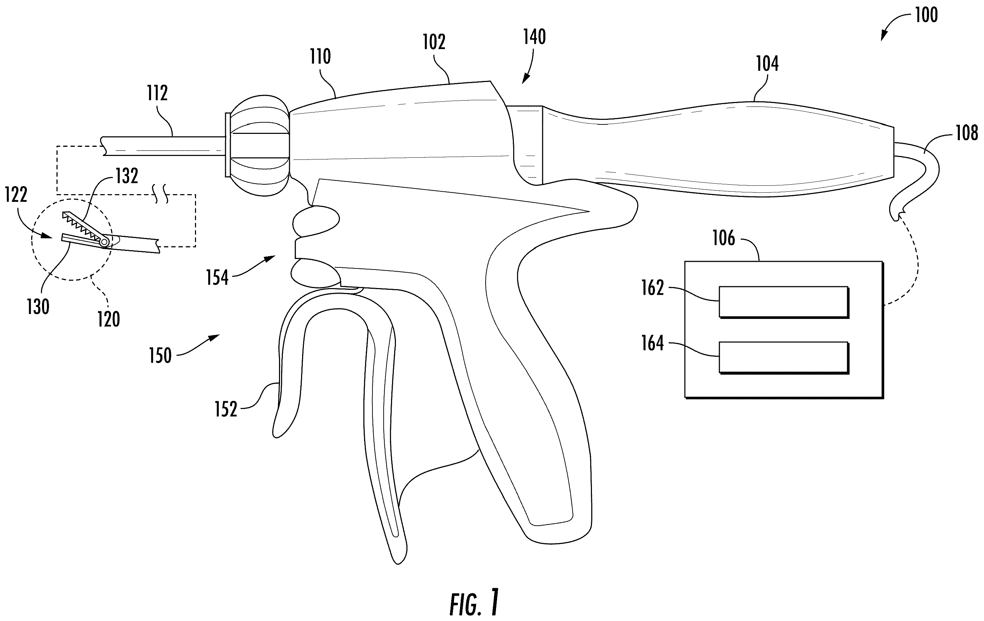

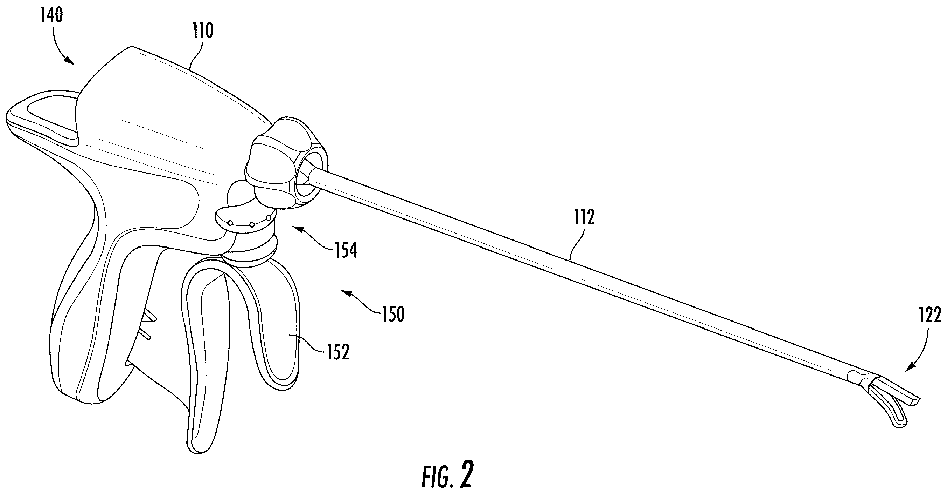

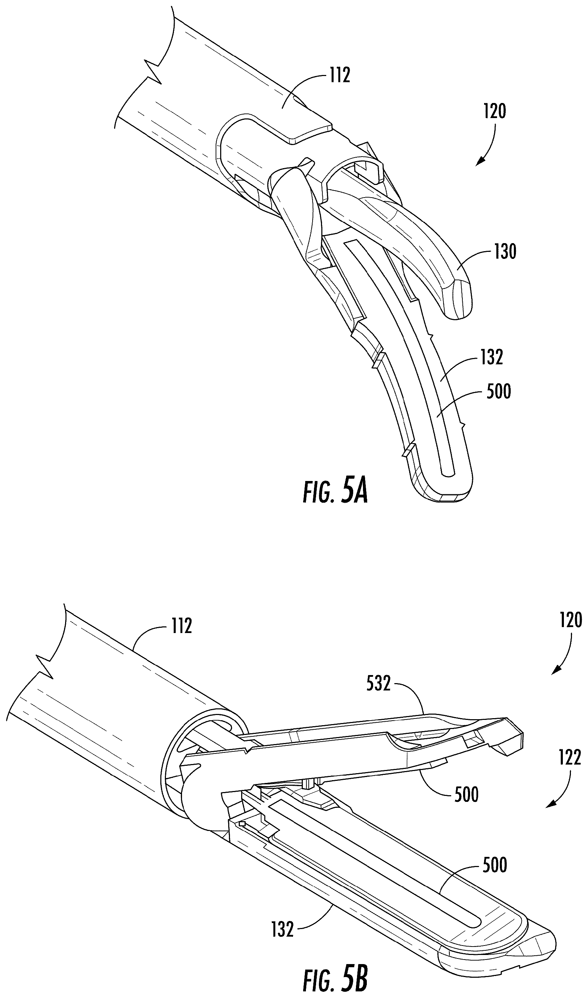

Referring now to FIGS. 1 and 2, in an illustrative embodiment, a system 100 for performing an energy-based surgical procedure includes a surgical instrument 102, a transducer 104, and a generator 106. The surgical instrument 102 is illustratively embodied as an ultrasonic surgical instrument, but may be embodied as an electro-surgical surgical instrument or a multi-modal, ultrasonic/elector-surgical surgical instrument in other embodiments. In use, the surgical instrument 102 is usable to perform various surgical procedures including laparoscopic, endoscopic, or traditional open surgical procedures. In doing so, a surgeon may selectively activate an ultrasonic mode (and/or an electro-surgical/RF mode) of the surgical instrument 102. In the ultrasonic mode, the generator 106 drives the transducer 104 to cause an ultrasonic blade 130 of a jaw assembly 122 of an end effector 120 of the surgical instrument 102 to vibrate at a reference frequency, which facilitates the contemporaneous cutting and hemostatic sealing of patient tissue. Additionally or alternatively, in some embodiments, the surgeon may selectively activate an electro-surgical mode of the surgical instrument 102 to deliver an amount of therapeutic RF energy to the patient tissue to effect hemostatic sealing. In such embodiments, the blade 130 may be embodied as an ultrasonic blade 130 or as a mechanical blade designed to cut tissue using mechanical force (e.g., in those embodiments not employing ultrasonic technologies). Furthermore, in some embodiments, the surgical instrument 102 may be configured with only an electro-surgical/RF mode and, in such embodiments, the jaw assembly 122 of the end effector 120 may not include the ultrasonic blade 130 as discussed in more detail below in regard to FIG. 5B.

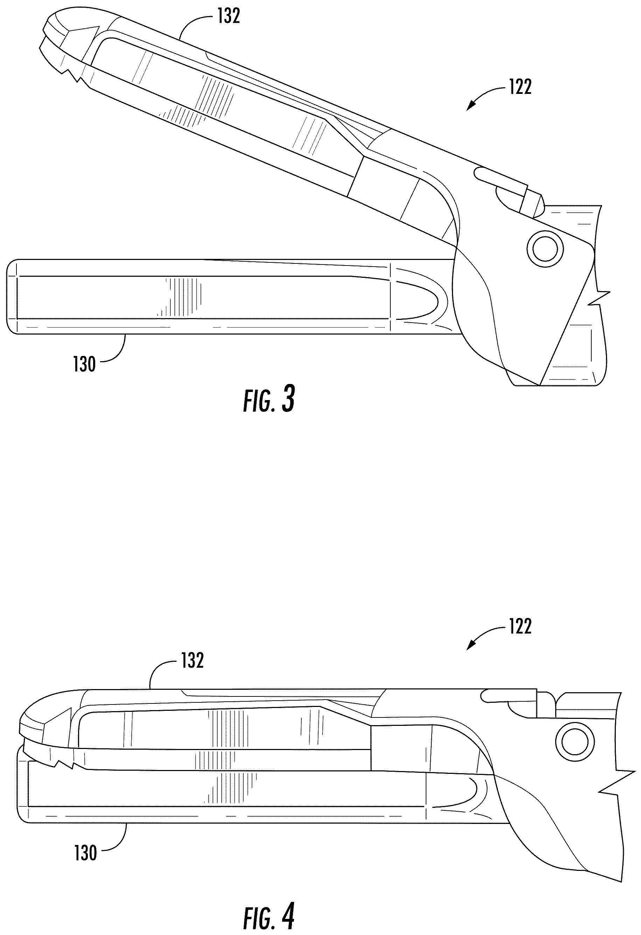

The surgical instrument 102 is illustratively embodied as ultrasonic surgical shears but may be embodied as other types of surgical instruments having an ultrasonic mode and/or electro-surgical mode in other embodiments. In the illustrative embodiment, the surgical instrument 102 includes a handle assembly 110 and an elongated shaft assembly 112, which extends distally away from the handle assembly 110 and may be removably attached to the handle assembly 110 in some embodiments. The elongated shaft assembly 112 includes the end effector 120 located at a distal end opposite the handle assembly 110. The end effector 120 includes the jaw assembly 122, which illustratively includes the ultrasonic blade 130 and a corresponding jaw clamp 132 (but may include two jaw clamps in those embodiments having only an electro-surgical/RF mode). As shown in FIGS. 3 and 4, the jaw assembly 122 is movable between an open state (FIG. 3) in which the jaw clamp 132 is positioned away from the ultrasonic blade 130 and a closed state (FIG. 4) in which the jaw clamp 132 is positioned near or otherwise contacts the ultrasonic blade 130. Actuation of the jaw assembly 122 from the open state to the closed state allows for the grasping, cutting, and coagulation of vessels and/or tissue by the jaw assembly 122. It should be appreciated that the open state may correspond to a degree of openness that is less than a fully opened position of the jaw assembly 122 and the closed state may correspond to a degree of closeness that is less than a fully closed position. That is, the closed state may, for example correspond to a minimal distance between the distal ends of the jaw clamp 132 and the ultrasonic blade 130 and the open state may correspond to a maximum distance between the distal ends of the jaw clamp 132 and the ultrasonic blade 130. However, in other embodiments, the open state may correspond to a fully opened position of the jaw assembly 122 and the closed state may correspond to a fully closed position of the jaw assembly 122.

In those embodiments in which the surgical instrument 102 includes both a ultrasonic mode and an electro-surgical/RF mode, the end effector 120 may include one or more RF electrodes 500 incorporated into the jaw clamp 132 as shown in FIG. 5A. Although the illustrative end effector 120 includes only a single electrode 500 in the embodiment of FIG. 5A, it should be appreciated that the end effector 120 may include additional electrodes 500 in other embodiments (e.g., multiple pads of electrodes 500). The electrode(s) 500 may be embodied as an active electrode configured to the RF energy or as a return electrode configured to "sink" an applied RF energy. In those embodiments utilizing bi-polar RF implementation, the ultrasonic blade 130 may embody the active or return electrode, with the electrode 500 embodying the other active or return electrode. Alternatively, other active or return electrodes may be incorporated on the ultrasonic blade 130 or in another part of the jaw assembly 122 of the end effector 120. In mono-polar implementation, the RF electrode(s) 500 may be embodied as an active electrode, and a return electrode may be attached to a portion of the patient's body.

In those embodiments in which the surgical instrument 102 includes only an electro-surgical/RF mode, the jaw assembly 122 of the end effector 120 includes a jaw clamp 532 in place of the ultrasonic blade 130 as shown in FIG. 5B. In such embodiments, an electrode 500 may be attached to or otherwise incorporated into each jaw clamp 132, 532 and be embodied as an active or a return electrode to facilitate the application of RF energy to tissue captured between the jaw clamps 132, 532. In such embodiments, the surgical instrument 102 may include a knife incorporated into the elongated shaft assembly 112 that is configured to eject outwardly to cut the patient's tissue after sealing of the tissue by the RF energy.

Referring back to FIGS. 1 and 2, in those embodiments including ultrasonic capabilities, the handle assembly 110 includes a receptacle 140 configured to receive the transducer 104 to facilitate connection of the transducer 104 to the handle assembly 110 and the elongated shaft assembly 112. The handle assembly 110 also includes a trigger assembly 150, which includes a primary trigger 152 and a switch assembly 154. The primary trigger 152 is operable by the surgeon to move the jaw assembly 122 of the end effector 120 between the open and closed states. The switch assembly 154 includes one or more buttons, which are selectable by the surgeon to activate (and configure, in some embodiments) the ultrasonic mode and/or the electro-surgical mode of the surgical instrument 102.

The transducer 104 is illustratively connected to the generator 106 by a cable assembly 108. As discussed above, the generator 106 is configured to drive the transducer 104 at a reference or resonant frequency to thereby cause the ultrasonic blade 130 to vibrate. For example, in an illustrative embodiment, the generator 106 may supply an electrical signal to the transducer 104 to cause the ultrasonic blade 130 of the jaw assembly 122 to vibrate longitudinally in the range of, for example, approximately 20 kHz to 250 kHz. In particular embodiments, for example, the ultrasonic blade 130 may vibrate in the range of about 54 kHz to 56 kHz (e.g., at about 55.5 kHz). In other embodiments, the ultrasonic blade 130 may vibrate at other frequencies including, for example, about 31 kHz or about 80 kHz. The excursion of the vibrations at the ultrasonic blade 130 can be controlled by, for example, controlling the amplitude of the electrical signal applied to the transducer 104 by the generator 106. The generator 106 may be activated so that electrical energy may be continuously or intermittently supplied to the transducer 104. The generator 106 also has a power line (not shown) for insertion in an electro-surgical unit or conventional electrical outlet. Additionally or alternatively, the generator 106 may be powered by a direct current (DC) source, such as a battery.

In some embodiments, the generator 106 may be configured to operate in different modes. In such embodiments, the generator 106 may include an ultrasonic generator module 162 for controlling an ultrasonic mode, an electro-surgical/Radio Frequency (RF) generator module 164 for controlling an electro-surgical mode, and/or other generator modules (e.g., a heat generator module) for controlling other operation modes. The various modes of the generator 106 may be operated independently of each other in some embodiments. For example, the generator 106 may activate the ultrasonic mode of the ultrasonic generator module 162 to apply ultrasonic energy to the jaw assembly 122 and subsequently, either therapeutic or sub-therapeutic RF energy may be applied to the jaw assembly 122 by the electro-surgical generator module 164. Alternatively, the activation modes of the generator 106 may be operated simultaneously or contemporaneously with each other.

In the electro-surgical mode, the electro-surgical generator module 164 is configured to generate RF energy at a frequency in the range of about 100 kilohertz (100 kHz) to about 1 megahertz (1 MHz). The generated RF energy is supplied to the patient's tissue via the electrodes 500 of the end effector 120 as described above in regard to FIG. 5. In some embodiments, the electro-surgical generator module 164 may also be configured to selectively provide the RF energy at sub-therapeutic levels to perform various electrical measurements of the patient's tissue. For example, the electro-surgical generator module 164 may be configured to measure an impedance of the patient's tissue using the electrodes 500 and a suitable RF energy level.

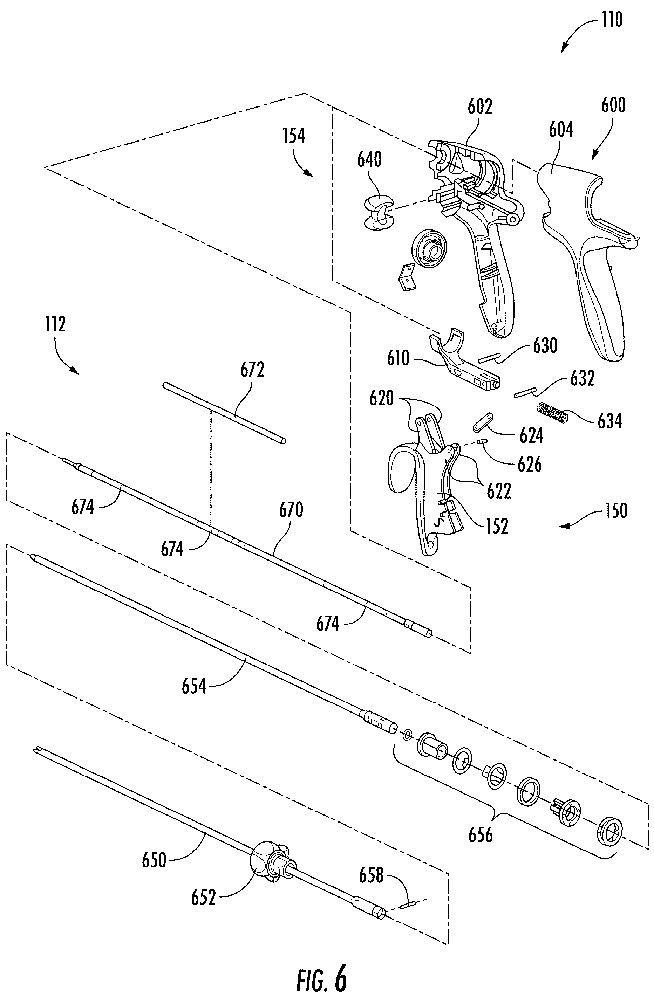

Referring now to FIG. 6, as discussed above, the illustrative surgical instrument 102 includes the handle assembly 110 and the elongated shaft assembly 112, which extends distally away from the handle assembly 110. The handle assembly 110 includes a housing 600, which includes a right half-housing 602 and a left half-housing 604. The half housings 602, 604 are configured to mate with each other to form the housing 600. To facilitate such mating, each of the half housings 602, 604 may include various interfaces sized to mechanically align and engage one another to form the housing 600 and enclose the internal working components of the surgical instrument 102.

The primary trigger 152 of the trigger assembly 150 is coupled to a linkage mechanism to translate the rotational motion of the primary trigger 152 to axial motion of a yoke 610, which in turn is configured to move the jaw assembly 122 of the end effector 120 between the open and closed states via the elongated shaft assembly 112. The primary trigger 152 includes a first set of flanges 620 having openings formed therein to receive a first yoke pin 630, which extends through the yoke 610. The primary trigger 152 also includes a second set of flanges 622 configured to receive a first end of a link 624. A trigger pin 626 is received in openings formed in the first end of the link 624 and the second set of flanges 622. The trigger pin 626 forms a trigger pivot point for the primary trigger 152. A second end of the link 624, opposite the first end, is received in a slot formed in a proximal end of the yoke 610 and retained therein by a second yoke pin 632. As the primary trigger 152 is rotated about the pivot point formed from the trigger pin 626, the yoke 610 translates horizontally. A spring 634 is used to bias the yoke forward such that the jaw assembly 122 of the end effector 120 is biased to the open state (or a fully opened state).

As discussed above, the trigger assembly 150 also includes a switch assembly 154. The switch assembly 154 illustratively includes a toggle switch 640, which is selectable to activate one or more switches 642. Activation of the switches 642 electrically energizes an electrical element 644, which electrically energizes the ultrasonic transducer 104 to engage the ultrasonic mode of the surgical instrument 102.

The elongated shaft assembly 112 includes an outer tubular sheath 650 and a rotation knob 652 coupled to the outer cylindrical sheath 650. The rotation knob 652 is operable to rotate the outer cylindrical sheath 650 about an axis defined by the outer cylindrical sheath 650. A reciprocating tubular actuator 654 is located within the outer tubular sheath 650 and mechanically engaged with the end effector 120 on a distal end. The reciprocating tubular actuator 654 is also mechanically engaged, on a proximal end, with the yoke 610 within the handle assembly 110 via coupling elements 656. In embodiments including an ultrasonic mode, an ultrasonic waveguide 670 is located within the reciprocating tubular actuator 654. A distal end of the ultrasonic waveguide 670 is acoustically coupled (e.g., directly or indirectly mechanically coupled) to the ultrasonic blade 130, and a proximal end is acoustically coupled to the transducer 104. The ultrasonic waveguide 670 may be isolated from other components of the elongated shaft assembly 112 by a protective sheath 672 and a number of isolation elements 674. The outer tubular sheath 650, the reciprocating tubular actuator 654, and the ultrasonic waveguide 670 are mechanically engaged together via a pin 658.

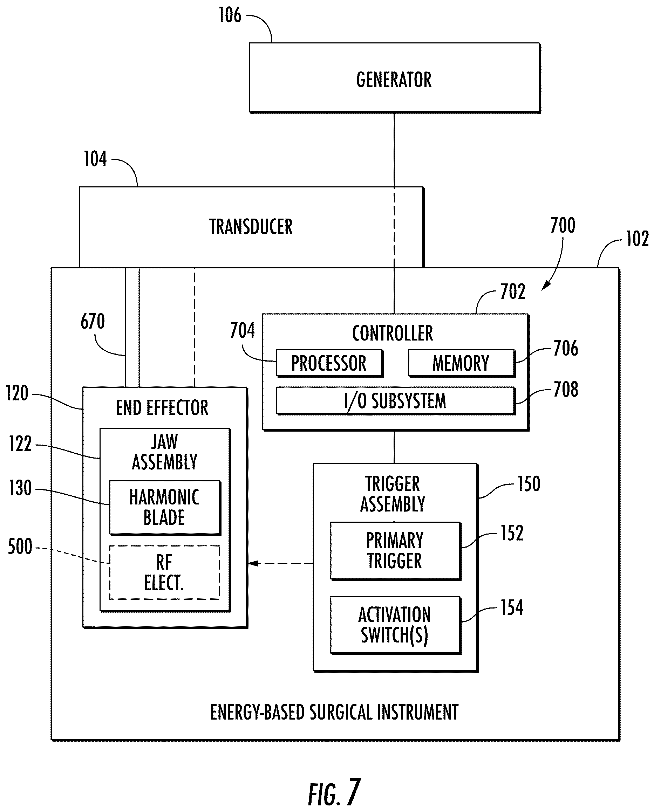

Referring now to FIG. 7, in the illustrative embodiment, the surgical instrument 102 includes a control circuit 700. The control circuit 700 includes a controller 702 and the trigger assembly 150, which cooperate to provide ultrasonic energy to the harmonic blade 130 of the jaw assembly 122 of the end effector 120 and/or RF energy to the RF electrodes 500 of the jaw assembly 122, depending on the operation modes of the surgical instrument 102 as discussed above. In other embodiments, however, the control circuit 700 may include additional or other electronic devices and/or circuit.

The controller 702 may be embodied as any type of controller, functional block, digital logic, or other component, device, circuitry, or collection thereof capable of performing the functions described herein. In illustrative embodiment, the controller 702 includes a processor 704, a memory 706, and an input/output (I/O) subsystem 708. The processor 704 may be embodied as any type of processor capable of performing the functions described herein. For example, the processor 704 may be embodied as a single or multi-core processor(s), digital signal processor, microcontroller, or other processor or processing/controlling circuit. Similarly, the memory 706 may be embodied as any type of volatile and/or non-volatile memory or data storage capable of performing the functions described herein. In operation, the memory 706 may store various data and software used during operation of the control circuit 700 such as executable firmware or software, programs, libraries, and drivers, which may be executed or otherwise used by the processor 704.

The processor 704 and memory 706 are communicatively coupled to other components of the control circuit 700 via the I/O subsystem 708, which may be embodied as circuitry and/or components to facilitate input/output operations between the controller 702 (e.g., the processor 704 and the memory 706) and the other components of the control circuit 700. For example, the I/O subsystem 708 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 708 may form a portion of a system-on-a-chip (SoC) and be incorporated, along with the processor 704 and the memory 706, and other components of the surgical instrument 102, on a single integrated circuit chip. Additionally, in some embodiments, the memory 706, or portions of the memory 706, may be incorporated into the processor 704.

During operation, as discussed above, the controller 702 is configured to control activation of an ultrasonic mode and/or an electro-surgical/RF mode of the surgical instrument 102. To do so, the controller 702 may monitor for activation of the primary trigger 152 and/or one or more activation switches 154 of the trigger assembly 150. In response to activation of the appropriate trigger 152 or switch 154, the controller 702 controls the transducer 104 to generate the ultrasonic energy, which is propagated to the harmonic blade 130 via the ultrasonic waveguide 670. Additionally or alternatively, in response to activation of a corresponding switch 154 of the trigger assembly 150, the controller 702 may be configured to supply an amount of RF energy, via the electro-surgical generator module 164 to the RF electrodes 500 via interconnections 710. It should be appreciated that, although the transducer 104 and the generator 106 are shown as separate components from the energy-based surgical instrument 102 in FIGS. 1 and 7, the transducer 104 and/or the generator 106 may be incorporated into the surgical instrument 102 in other embodiments.

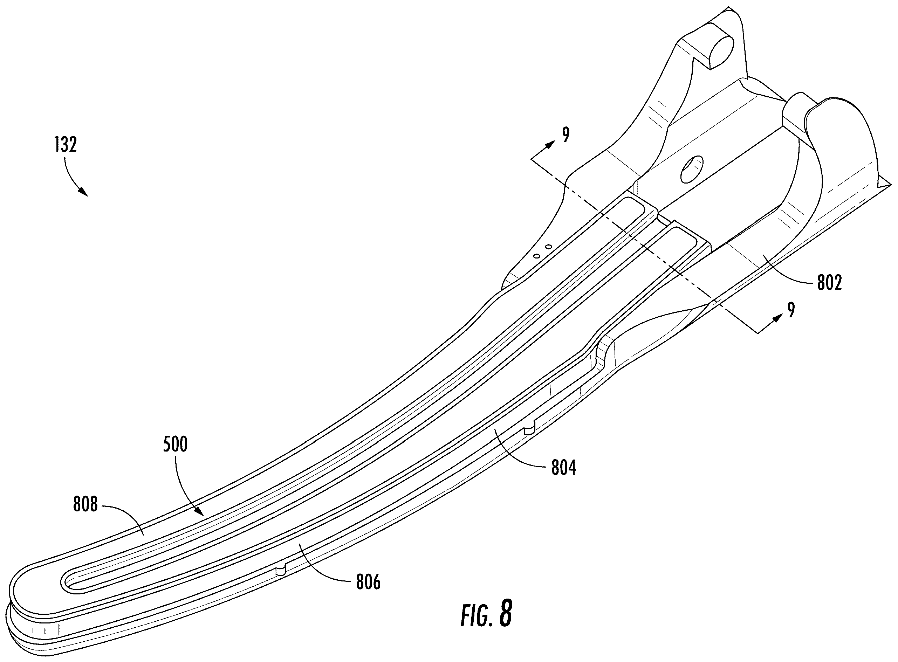

Referring now to FIG. 8, in another embodiment, the jaw clamp 132 is fabricated to include an elongated metallic body 802 and a non-stick tissue pad 804. The tissue pad 804 includes a polymeric substrate 806, which is formed from polytetrafluoroethylene (PTFE). PTFE is a solid fluoropolymer that is chemically inert and has a very low coefficient of friction. Accordingly, the PTFE substrate 806 may exhibit reduced sticking with tissue and other material. In use, when the jaw assembly 122 is actuated from the open state to the closed state, the jaw assembly 132 and thus the tissue pad 804 may contact and otherwise grip tissue and/or blood vessels. In some embodiments, the tissue pad 804 may include one or more tissue stability features such as teeth, posts, ridges, or other features (not shown), which may be formed in the PTFE substrate 806.

As discussed above, in the illustrative embodiment, the polymeric substrate 806 is formed from PTFE. It should be understood that in other embodiments, the substrate 806 may be formed from one or more other relatively hard polymers, including but not limited to polyetheretherketone (PEEK), polyetherimide (PEI), polyphenylene sulfide (PPS), polyimide (PI) polysulfone (PSU), polypropylene (PP), polystyrene (PS), polyethylene terephthalate (PET), fiberglass (FG), polycarbonate (PC), acrylonitrile butadiene styrene (ABS), and/or polyvinylchloride (PVC).

The tissue pad 804 further includes a conductive trace 808 formed on the PTFE substrate 806. The conductive trace 808 may be formed from metallic material, conductive ink, conductive polymer, or other conductive material. The conductive trace 808 may be embodied as an RF electrode 500 such as an active electrode configured to apply RF energy as described above. Accordingly, in some embodiments, in an electro-surgical mode, generated RF energy may be supplied to the patient's tissue by the conductive trace 808. The RF energy may be returned by a return electrode such as the ultrasonic blade 130. The RF energy may be provided at therapeutic or sub-therapeutic levels.

Accordingly, the illustrative tissue pad 804 including the conductive trace 808 may be used for a single energy-based surgical instruction 102 for both ultrasonic cutting and advanced bi-polar sealing (e.g., electro-surgical or RF sealing). Thus, the conductivity of the PTFE pad (i.e., the conductive trace 808) may be only in a specific area of a specific shape to prevent contact of RF energy with ultrasonic energy. Such an instrument 102 capable of both ultrasonic and RF energy-based cutting and sealing may improve surgical efficiency. For example, minimally invasive surgical procedures involve precise cutting and sealing of tissue, which are typically performed using multiple instruments. Instrument exchange (i.e., more than one instrument) in an operating room may cause delay and distraction as surgeons transition between reliable hemostasis and precise dissection devices, which may be avoided with an instrument 102 as described herein capable of both ultrasonic and RF operation.

The instrument 102 described herein allows both an RF and ultrasonic system to work together, because the ultrasonic blade 130 (i.e., a conductive metallic structure) is provided a PTFE pad 804 for support to generate the frictional mechanical energy that makes heat for tissue welding, and the RF system is provided the ultrasonic blade 130 and the conductive trace 808 as separate, non-shorted electrodes (e.g., return path and originating path electrodes) that are not shorted by any direct metallic contact, but are within 0.003-0.01" apart, which allows for the RF energy to transmit through the tissue, generating heat. Accordingly, there are portions of the PTFE pad 804 that are non-conductive so the ultrasonic blade 130 has contact but no shorting, and portions of the pad 804 that are conductive but don't touch the blade 130. Thus, the arrangement of the conductive trace 808 on the pad 804 may provide both conductive and specifically non-conductive portions to insure the proper operating of both energy forms (e.g., RF and ultrasound) of the instrument 102.

When the jaw assembly 122 is in the closed state, the ultrasonic blade 130 may be in contact with part or all of the PTFE substrate 806 of the tissue pad 804. Accordingly, the tissue pad 804 may be used as a support/pressure application structure to the ultrasonic blade 130. When the ultrasonic blade 130 is activated and thereby vibrates, the ultrasonic blade 130 may erode material from the tissue pad 804. As such, the conductive trace 808 and/or the PTFE substrate 806 may be arranged such that, as the tissue pad 804 erodes, the ultrasonic blade 130 does not contact the conductive trace 808, remains electrically insulated from the conductive trace 808, does not short-circuit with the conductive trace 808, or otherwise maintains an electrical relationship with the conductive trace 808, even when the tissue pad 804 is too metallicized for the ultrasonic blade 130 to erode through. Accordingly, in those embodiments, the instrument 102 may be used in the electro-surgical mode (supplying RF energy) even after operation in the ultrasound mode has eroded the tissue pad 804.

In some embodiments, the conductive traces 808 may be formed from multi-layer laminate conductive traces 808. The thickness of the conductive traces 808 could make it necessary for the conductive portion in one of more layers, enabling the ultrasonic blade 130 to erode into the trace 808 while maintaining the conductivity. The trace 808 could also be interconnected across the blade contact area, enabling the first activation the blade 130 to cut through the conductive trace 808 and leaving a receiving groove in the PTFE substrate 806 for the blade 130 to contact with the remaining RF electrode being perfectly aligned to the opening in the remaining printed electrode since it created it.

For example, in some embodiments the tissue pad 804 may include conductive ink electrodes having a portion that is eroded by the ultrasonic blade 130 to prevent blade-to-electrode continuity during RF application. In this embodiment, the ultrasonic blade 130 motion may cut a wider opening in the conductive surface 808 due to the blade's motion, which produces an electrode that will not short to the ultrasonic blade 130 return in RF mode. The temperature of the blade 130 in its cut-in configuration may move enough PTFE to create surrounding barriers to RF direct shorting between the blade 130 and the remaining conductive trace 808. Continuing that example, and referring to the illustrative tissue pad 804 shown in FIG. 8, in an embodiment the conductive trace 808 may cover the entire upper surface of the PTFE substrate 806, without including any central channel or relief for the ultrasonic blade 130. Continuing that example, the first ultrasonic activation may destroy the portion of the conductive trace 808 and the PTFE substrate 806 that makes the central non-conductive trough. After this first ultrasonic activation, the tissue pad 804 would look the same as shown in FIG. 8, but as described, the first non RF activation could be used to eliminate the portions of the conductive trace 808 that would otherwise short when the RF is activated.

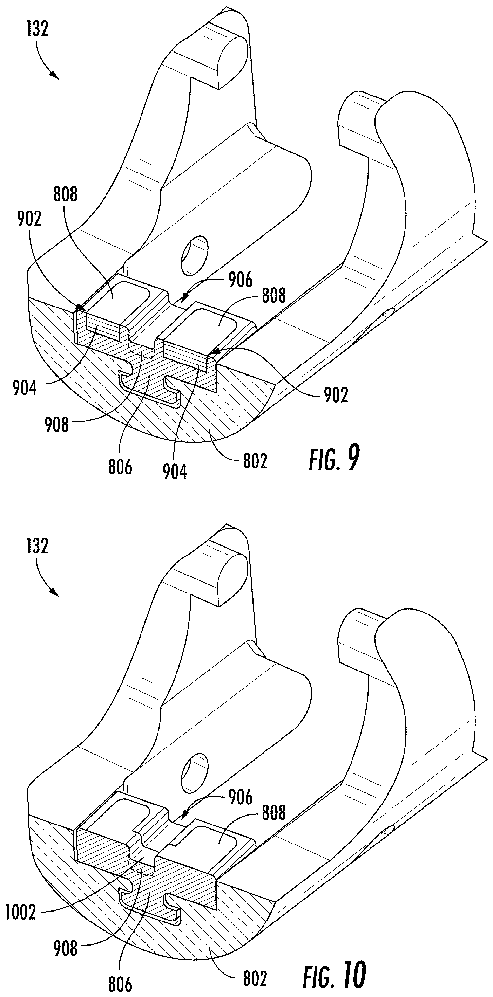

Referring now to FIG. 9, an illustrative embodiment of a jaw clamp 132 coupled to a tissue pad 804 is shown in cross-section. As shown, a pair of recesses 902 are defined in the tissue pad 804. The recesses 902 extend downward into the PTFE substrate 806. The conductive trace 808 fills each of the recesses 902. As shown, the conductive trace 808 is illustratively formed from multiple conductive layers 904. Accordingly, the illustrative trace 808 is a multi-layer laminate conductive trace. In the illustrative embodiment, part of the thickness of the trace 808 (e.g., one or more layers 904) may be eroded by the ultrasonic blade 130, and after erosion, additional thickness of the trace 808 (e.g., additional one or more layers 904) remains. This additional thickness of the trace 808 and/or additional layers 904 may be used provide RF energy for RF activation.

In some embodiments, the ultrasonic blade 130 may contact the tissue pad 804 within a channel 906 defined between parts of the conductive trace 808. When the ultrasonic blade 130 is activated, the ultrasonic blade 130 may erode the PTFE substrate 806 within the channel 906. For example, the surface of the PTFE substrate 806 may be eroded down to eroded level 908 after activation of the ultrasonic blade 130. After erosion, the conductive trace 808 remains electrically insulated and/or isolated from the ultrasonic blade 130.

Referring now to FIG. 10, another illustrative embodiment of a jaw clamp 132 coupled to a tissue pad 804 is shown in cross-section. As shown, in the illustrative embodiment of FIG. 10, the conductive trace 808 includes a bridge portion 1002 that crosses across the channel 906. In other words, the bridge portion 1002 of the conductive trace 808 extends across a part of the tissue pad 804 that contacts the ultrasonic blade 130. When the ultrasonic blade 130 is activated in ultrasonic mode, the tissue pad 804 (including the bridge portion 1002 of the trace 808 and the PTFE substrate 806) is eroded away, for example down to the eroded level 908. After erosion, the bridge portion 1002 has been removed and no longer contacts the ultrasonic blade 130 when the blade 130 contacts the tissue pad 804. Accordingly, after erosion, the conductive trace 808 remains electrically insulated and/or isolated from the ultrasonic blade 130.

In some embodiments, the conductive trace 808 may also be a multilayer laminate structure in the blade contact area (e.g., within the channel 906). In those embodiments, even some thickness of the conductive trace 808 remains within the blade contact area after erosion, the trace 808 may remain electrically insulated and/or isolated from the ultrasonic blade 130. For example, motion of the ultrasonic blade 130 may erode an opening in the tissue pad 804 (and the conductive trade 806) that is wider than the blade 130 itself, thereby ensuring that the trace 808 will not short to the ultrasonic blade 130 return in RF mode. As another example, the temperature of the ultrasonic blade 130 may melt and/or otherwise move enough of the PTFE substrate 806 to form surrounding barriers to prevent direct shorting between the ultrasonic blade 130 and the conductive trace 808.

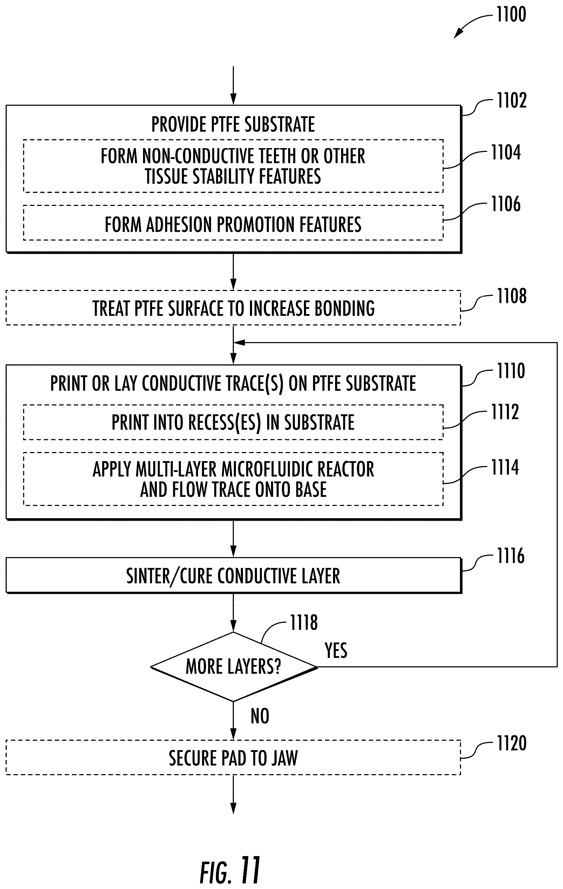

Referring now to FIG. 11, a method 1100 for manufacturing and assembly of a tissue pad 804 with the surgical instruction 102 is shown. The method 1100 begins in block 1102, in which a polytetrafluoroethylene (PTFE) substrate 806 is provided. The PTFE 806 may be molded, machined, or otherwise formed into an appropriate shape for use with the surgical instrument 102.

In some embodiments, in block 1104, non-conductive teeth or other tissue stability features may be formed in the surface of the PTFE substrate 806. In some embodiments, the non-conductive teeth may be 3D printed or otherwise formed on the surface of the PTFE substrate 806. In some embodiments, the non-conductive tissue stability features may be formed from PTFE or another polymer. The non-conductive tissue stability features may be formed as cylinders with sharpened points, cylinders with lateral surfaces (similar to certain anatomical teeth), or other tissue grasping features. In some embodiments, the electrode gap between the electrode and the return path metal (e.g., between the conductive trace 808 and the blade 130) may be very small, for example within the range of 0.003-0.015" or within the range of 0.005-0.01". Non-conductive teeth or other stability features may maintain that minimum gap, preventing shorting. Thus, the selectively conductive tissue pad 804 includes certain selective portions of the pad 804 that are conductive, doing one job (e.g., the conductive trace 808) and adjacent portions of the pad doing another job (e.g., the non-conductive teeth or tissue stability features).

In some embodiments, in block 1106, adhesion promotion features may be formed in the surface of the PTFE substrate 806. For example, the adhesion promotion features may include geometry such as short but proud features in the PTFE substrate 806 that the conductive trace is printed around and over. This would provide larger geometric features for resisting lateral delamination of the electrode circumferentially from the PTFE 806. These standing features could also be printed circumferentially around the intended ultrasonic blade 130 interaction location on the PTFE substrate 806. This may give the blade 130 a location to seat onto, and the melted/eroded PTFE may create a further circumferential barrier to unwanted RF shorting after the eroding has begun.

In some embodiments, in block 1108, the surface of the PTFE substrate 806 may be treated to increase bonding with other materials, such as the conductive trace 808. The surface may be prepared by mechanically roughening the surface, chemically etching the surface with sodium ammonia, plasma etching the surface, coating the surface with parylene, or another surface preparation technique. In some embodiments, one or more of the surface treatments may be combined to achieve a synergistic effect. For example, in an embodiment, sodium ammonia etching, plasma treatment, and parylene coating may be performed to the surface of the PTFE substrate 806. In some embodiments, the surface of the PTFE substrate 806 may be modified or prepared to a depth of 10 µm or less, for example through plasma or reactive etching. Additionally or alternatively, in some embodiments, the surface of the PTFE substrate 806 may be roughened to a depth of at least 30 µm, for example by mechanical roughening. In some embodiments, the surface of the PTFE substrate 806 may be modified to a depth within a range of about 5-30 µm, or in some embodiments, within a range of about 5-10 µm. The surface roughness range may depend on the method used in creating the conductive layer. For example, if soft lithography is used, the finish may be down to 5-10 µm and still have good adhesion, using 3-10 µm thick traces. As another example, if more conventional 3D printing is used, the surface may have closer to 30 µm in roughness. This roughness, desired for electrode adhesion, is counter to the slick or smooth properties of the PTFE that is desired for tissue friction and ultrasonic blade interface.

Typcally, PTFE material is developed to have a low surface energy to prevent materials sticking on to its surface. To make the PTFE material more receptive to conductive material trace adhesion, several surface treatment options (such as argon plasma treatment, parylene coating, sodium ammonia etching, or other options) are possible to decrease surface energy. In an embodiment, sodium ammonia surface etching decreases surface fluorine on the PTFE substrate 806 for an extended time and permitted chemical and mechanical bonding. In embodiment, sodium ammonia surface etching may be preferred over plasma treatment and parylene coating.

In block 1110, one or more conductive traces 808 are printed or otherwise laid on the surface of the PTFE substrate 806. The conductive trace 808 may be formed from conductive ink, conductive polymer, metallic material, or other conductive material. The conductive traces 808 are adhered to the PTFE substate 806 so as to resist delamination. For example, the conductive traces 808 may be deposited via a 3D micro-dispensing process onto a sodium ammonia etched PTFE substrate 806 with a fine resolution. The resulting trace 808 is conductive and may meet the geometry needs of the medical device. Additionally, all elements deposited on the PTFE substrate 806 may be low-profile on the device to be minimally invasive and biocompatible. As another example, the conductive trace 808 may be 3D printed on the surface of the substrate 806.

In some embodiments, in block 1112, the conductive trace 808 may be printed into one or more recesses in the PTFE substrate 806. As described above, in some embodiments those recesses may be formed in one or more tissue stability features. Accordingly, when the conductive trace 808 fills those recesses, an electrode may be formed having a gripping aspect of the manipulation of tissue. Additionally or alternatively, a selectively conductive path within a non-conductive PTFE teeth pad geometry may be formed.

In some embodiments, in block 1114, a multi-layer microfluidic reactor may be applied to the PTFE substrate 806. The conductive trace 808 is then flowed through the microfluidic reactor onto the base (i.e., the PTFE substrate 806). Alternatively soft, multi-layered stretchable microfluidic reactors may be used as a bounding layer, which takes advantage of the compressibility of elastomeric materials to create reversible seals. The conductive traces 808 are then deposited directly onto flat polymer surfaces with textured finishes, relief patterns, as well as those with 3D features/geometries via localized, flow-assisted, solution-phase electroless deposition. Accordingly, desired patterns of conductive traces 808 can be generated through the rational design of the channel network within the soft reactors, and necessary additions to the patterned traces 808 that are needed to complete a circuit can be achieved by reorienting the soft micro-reactor (or reactors of different designs) for successive depositions (or etching steps). In some embodiments, the soft multi-layer surface could be made of stiff silicone (e.g., polydimethylsiloxane, PDMS) and a soft silicone (e.g., Ecoflex^{™}) using 3D printing and soft lithography. This technique could be used to adhere traces on harder polymers, such as teflon (PTFE), polyetheretherketone (PEEK), polyetherimide (PEI), polyphenylene sulfide (PPS), polyimide (PI) polysulfone (PSU), polypropylene (PP), polystyrene (PS), polyethylene terephthalate (PET), fiberglass (FG), polycarbonate (PC), acrylonitrile butadiene styrene (ABS), and polyvinylchloride (PVC). In an embodiment, 3-10 µm traces could be applied using a 1-5 µm thick reactor. The microfluidic channels could be created in the base polymer, and the multi-layer reactor placed into the channels, thus creating their own sub channels to receive the conductive traces. This would enable finished traces down to 5-10 µm and still maintain adherence.

In block 1116, the conductive trace 808 is sintered, cured, or otherwise hardened or finalized using any appropriate technique. This sintering or curing may allow the conductive trace 808 to adhere to and otherwise stay on the PTFE substrate 806 while the conductive trace 808 goes through multiple RF or other energy activation cycles and remains conductive. For example, in an embodiment, the conductive trace 808 may remain attached to the PTFE substrate 806 for more than 100 RF energy activation cycles.

In block 1118, it is determined whether additional layers of the conductive trace 808 should be formed. If so, the method 900 loops back to block 1110, in which additional layers of the conductive trace 808 are deposited as described above. Accordingly, the conductive trace 808 may be embodied as a multi-layer laminated electrode, which may be erodible as described above. Additionally or alternatively, in some embodiments additional layers of other materials (e.g., non-conductive layer or other layers) may be deposited. For example, alternating conductive and non-conductive layers may be deposited and/or another arrangement of layers may be deposited. Referring again to block 1118, if no additional layers are to be formed, the method 900 advances to block 1120.

In block 1120, in some embodiments the tissue pad 804 may be secured to the jaw clamp 132. Of course, in some embodiments the tissue pad 804 may already be secured to the jaw clamp 132 prior to deposition of the conductive trace 808. The tissue pad 804 may be secured using any appropriate fixation technique. For example, in an embodiment, a tab extending from a bottom surface of the PTFE substrate 806 may be mechanically received and retained in a channel formed in the jaw clamp 132 as illustrated in FIGS. 9-10.

After electrically securing the pad 804 to the jaw clamp 132, the method 900 is completed. The completed assembly of the jaw clamp 132 and the tissue pad 804 with conductive trace 808 may be incorporated into a surgical instrument 102 and used as described above. Accordingly, the conductive trade 808 may be electrically coupled to the generator 106, for example with one or more wires, flex circuits, and/or other electrical circuits.

While the disclosure has been illustrated and described in detail in the drawings and foregoing description, such an illustration and description is to be considered as illustrative and not restrictive in character, it being understood that only illustrative embodiments have been shown and described and that all changes and modifications that come within the spirit of the disclosure are desired to be protected.

There are a plurality of advantages of the present disclosure arising from the various features of the methods, apparatuses, and systems described herein. It will be noted that alternative embodiments of the methods, apparatuses, and systems of the present disclosure may not include all of the features described yet still benefit from at least some of the advantages of such features. Those of ordinary skill in the art may readily devise their own implementations of the methods, apparatuses, and systems that incorporate one or more of the features of the present invention and fall within the spirit and scope of the present disclosure as defined by the appended claims.

## Claims

1. A surgical instrument comprising:
an end effector having an ultrasonic blade, a metallic jaw clamp, and a partially conductive tissue pad coupled to the jaw clamp;
wherein the tissue pad comprises a polymeric substrate with a conductive trace formed on the surface of the polymeric substrate, wherein the conductive trace covers part or all of the polymeric substrate.

2. The surgical instrument of claim 1, wherein the polymeric substrate comprises polytetrafluoroethylene (PTFE).

3. The surgical instrument of claim 1 or claim 2, wherein the surface of the polymeric substrate is treated to improve adhesion.

4. The surgical instrument of claim 3, wherein the surface of the polymeric substrate is treated by sodium ammonia etching, plasma treatment, and parylene coating.

5. The surgical instrument of any preceding claim, wherein the polymeric substrate comprises polyetheretherketone (PEEK), polyetherimide (PEI), polyphenylene sulfide (PPS), polyimide (PI) polysulfone (PSU), polypropylene (PP), polystyrene (PS), polyethylene terephthalate (PET), fiberglass (FG), polycarbonate (PC), acrylonitrile butadiene styrene (ABS), or polyvinylchloride (PVC).

6. The surgical instrument of any preceding claim, wherein the conductive trace comprises one of a conductive ink and a multi-layer laminate conductive trace.

7. The surgical instrument of claim 6, wherein the conductive trace comprises a multi-layer laminate conductive trace and wherein the multi-layer laminate conductive trace is erodible by the ultrasonic blade without electrically shorting to the ultrasonic blade, preferably wherein the multi-layer laminate conductive trace has a first thickness, and wherein the ultrasonic blade erodes less than the first thickness of the multi-layer laminate conductive trace when activated.

8. The surgical instrument of any preceding claim, wherein:
the end effector further comprises a jaw assembly movable between an open state and a closed state; and
when the jaw assembly is in the closed state, the metallic jaw clamp is clamped against the ultrasonic blade, and when clamped the polymeric substrate contacts the ultrasonic blade and the conductive trace is electrically insulated from the ultrasonic blade.

9. The surgical instrument of claim 8, wherein when the ultrasonic blade is activated the polymeric substrate erodes and the conductive trace remains electrically insulated from the ultrasonic blade.

10. The surgical instrument of any preceding claim, wherein:
the end effector further comprises a jaw assembly movable between an open state and a closed state;
when the jaw assembly is in the closed state, the metallic jaw clamp is clamped against the polymeric substrate, and when clamped the polymeric substrate contacts the ultrasonic blade at a blade contact area;
a first portion of the conductive trace extends across the blade contact area of the polymeric substrate and contacts the ultrasonic blade when the metallic jaw clamp is clamped against the polymeric substrate; and
when the ultrasonic blade is activated the first portion of the conductive trace erodes, and the conductive trace is electrically insulated from the ultrasonic blade after activation of the ultrasonic blade.

11. A method for constructing a surgical instrument, the method comprising:
forming a conductive trace on a surface of a polymeric substrate, wherein the trace covers part or all of the polymeric substrate; and
securing the polymeric substrate to a metallic jaw clamp of a surgical instrument.

12. The method of claim 11, wherein the polymeric substrate comprises polytetrafluoroethylene (PTFE).

13. The method of claim 11 or claim 12, wherein forming the conductive trace on the surface of the polymeric substrate comprises printing the conductive trace with one of a conductive ink and forming a multi-layer laminate conductive trace on the surface of the polymeric substrate.

14. The method of claim 13, wherein the conductive trace comprises a multi-layer laminate conductive trace and wherein the multi-layer laminate conductive trace is erodible by an ultrasonic blade without electrically shorting to the ultrasonic blade.

15. The method of any one of claims 11 to 14, further comprising clamping the metallic jaw clamp against an ultrasonic blade, wherein when clamped the polymeric substrate contacts the ultrasonic blade and the conductive trace is electrically insulated from the ultrasonic blade, optionally wherein when the ultrasonic blade is activated the polymeric substrate erodes and the conductive trace remains electrically insulated from the ultrasonic blade.

16. The method of any one of claims 11 to 15, further comprising clamping the metallic jaw clamp against an ultrasonic blade, wherein when clamped the polymeric substrate contacts the ultrasonic blade at a blade contact area, and wherein a first portion of the conductive trace extends across the blade contact area of the polymeric substrate and contacts the ultrasonic blade.
